# FASCICULE DE BREVET EUROPEEN

(11) **EP 2 200 659 B1**
(45) Date de publication et mention de la délivrance du brevet: **22.07.2015**
(21) Numéro de dépôt: 08843053.3
(22) Date de dépôt: 14.10.2008
(51) Int. Cl.: A61K 9/00, A61K 41/00, A61P 35/00, A61K 9/51, A61K 103/34, A61K 103/36

(54) **UTILISATION DE NANOPARTICULES A BASE DE LANTHANIDES COMME AGENTS RADIOSENSIBILISANTS**
VERWENDUNG VON NANOPARTIKELN AUF LANTHANIDBASIS ALS RADIOSENSITIZER
USE OF LANTHANIDE-BASED NANOPARTICLES AS RADIOSENSITIZING AGENTS

(30) Priorité: 16.10.2007 FR 0758348
(43) Date de publication de la demande: 30.06.2010
(73) Titulaire: Université Claude Bernard Lyon I, 69622 Villeurbanne Cedex (FR); Hospices Civils de Lyon, 69229 Lyon Cedex 02 (FR); Centre National de la Recherche Scientifique, 75794 Paris Cedex 16 (FR); Nano-H, 69001 Lyon (FR); European Synchrotron Radiation Facility (Installation Europeenne De Rayonnement Synchrotron), 38000 Grenoble (FR)
(72) Inventeur: TILLEMENT, Olivier, F-69270 Fontaines Saint Martin (FR); ROUX, Stéphane, F-69100 Villeurbanne (FR); PERRIAT, Pascal, F-69001 Lyon (FR); LEDUC, Géraldine, F-38920 Crolles (FR); MANDON, Céline, F-69006 Lyon (FR); MUTELET, Brice, F-69100 Villeurbanne (FR); ALRIC, Christophe, F-69500 BRON (FR); BILLOTEY, Claire, F-69003 Lyon (FR); JANIER, Marc, F-69400 LIMAS (FR); LOUIS, Cédric, F-38138 Les Cotes D'arey (FR)
(74) Mandataire: Sarlin, Laure V.
(86) Numéro de dépôt international: PCT/FR2008/051860
(87) Numéro de publication internationale: WO 2009/053644

(56) Documents cités:
- WO-A-03/080743
- WO-A-2005/120590
- WO-A-2006/012201
- FR-A- 2 863 053
- FR-A- 2 867 180
- LI W P ET AL: "Development of an in vitro model for assessing the in vivo stability of lanthanide chelates" NUCLEAR MEDICINE AND BIOLOGY, ELSEVIER, NY, US, vol. 28, no. 2, février 2001 (2001-02), pages 145-154, XP004233299 ISSN: 0969-8051
- HU F ET AL: "Pm-149 DOTA bombesin analogs for potential radiotherapy - in vivo comparison with Sm-153 and Lu-177 labeled DO3A-amide-betaAla-BBN(7-14)NH" NUCLEAR MEDICINE AND BIOLOGY, ELSEVIER, NY, US, vol. 29, no. 4, mai 2002 (2002-05), pages 423-430, XP004357345 ISSN: 0969-8051
- BAZZI R ET AL: "Synthesis and luminescent properties of sub-5-nm lanthanide oxides nanoparticles" JOURNAL OF LUMINESCENCE, AMSTERDAM, NL, vol. 102-103, mai 2003 (2003-05), pages 445-450, XP004416620 ISSN: 0022-2313 cité dans la demande

## Description

L'invention concerne de nouveaux agents destinés à augmenter l'efficacité des radiothérapies.

Plus précisément l'invention vise l'utilisation de nanoparticules à forte concentration en oxydes de lanthanides, comme radio-sensibilisants.

Les radiations X ou gamma sont largement utilisées pour traiter les tumeurs. Néanmoins, de telles radiations ne sont généralement pas spécifiques des tumeurs, et des doses de radiations importantes peuvent être également données à des tissus sains lors des traitements.

L'utilisation de radio-sensibilisants est proposée depuis de nombreuses années. Un composé radio-sensibilisant est un composé qui agit en combinaison avec la radiation pour induire une réponse plus efficace et augmenter l'efficacité thérapeutique. On vise généralement des composés qui incluent en leur sein des éléments lourds à numéro atomique élevé et qui interagissent directement avec la radiation en augmentant sa probabilité d'interaction, en créant des dommages aux cellules visées.

Une partie plus importante de l'énergie irradiée est alors absorbée et déposée localement autour des radio-sensibilisants et peut produire des électrons secondaires, électrons Auger, électrons Compton, ionisations, photons, radicaux libres par exemple, ou même simplement une élévation thermique.

Pour les thérapies du cancer, un objectif est d'augmenter localement la dose à la tumeur, les radio-sensibilisants doivent ainsi être accumulés préférentiellement dans les tumeurs par rapport aux tissus sains.

L'augmentation de dose d'irradiation X absorbée en raison de l'utilisation d'éléments à numéro atomique élevé est connue depuis plus de 50 ans et est considérée comme potentiellement intéressante pour augmenter les effets thérapeutiques in vitro (H. Matsudaira et al. « Iodine contrats médium sensitizes cultured mamallian cells to X-rays », Rad. Res. 84: 144-148, 1980). Des calculs et mesures précises de l'effet ont été réalisés par Das et al. (Das et al. « Backscatter dose perturbation in kilovoltage photon beams at high atomic number interfaces » Med. Phys. 22 : 767-773, 1995).

Des composés moléculaires utilisant des éléments lourds ont été proposés.

Par exemple le carboplatine, le cis platine et l'oxyplatine ont montré de bonnes efficacités (C. Diointe et al., « Comparisons of carboplatin and cisplatin as potentiators of 5-fluoroucacil and radiotherapy in mouse L1210 leukemia model » Anticancer Res. 22, 721-725, 2002).

Egalement des composés à base de lanthanides comme le gadolinium ou le lutetium ont été proposés. Deux metallotexaphyrines, la motexafine de gadolinium (Gd-Tex) et la motexafine de lutetium (Lu-Tex) font l'objet de développements importants (E. Kowinsky, oncology 13 (1999) 61)).

La Gd-Tex a en particulier la capacité d'induire un rehaussement à la fois du contraste en IRM et de l'effet de la dose délivrée.

Cependant il s'avère difficile de concentrer dans une tumeur ces agents en raison de leur caractère moléculaire qui favorise une élimination rapide et une diffusion extra-vasculaire. Cette caractéristique s'oppose à une application efficace de ces agents thérapeutiques car leur grande mobilité ne permet pas d'atteindre un différentiel suffisant entre la quantité d'agents dans la tumeur et celle dans les tissus sains. Cette répartition quasi homogène n'est pas sans risque car elle affecte autant les tissus sains que les tissus à traiter (sans compter la toxicité inhérente à certains de ces agents thérapeutiques).

L'utilisation de solides finement divisés a été proposée comme agents radio-sensibilisants pour pallier les insuffisances constatées avec les agents moléculaires.

L'utilisation de métal, particulièrement d'or, a semblé rapidement interressante. Ainsi Herold et al. ont proposé en 2000 l'utilisation de microbilles d'or (D. Herold et al., Gold microspheres a sélective technique for producing bilogically effective dose enhancement » Int. J. Rad. Biol. 76, 1357-1364, 2000) : la mobilité et la dispersion des poudres ont cependant rapidement posé problème.

Plus récemment, Hainfeld et al. ont proposé d'utiliser des nanoparticules métalliques pour augmenter l'effet thérapeutique (WO 2004/112590).

Il est proposé dans la demande internationale ci-dessus d'utiliser des nanoparticules métalliques composées d'éléments lourds, avec un grand numéro atomique Z, en particulier de l'or. L'utilisation de petites particules permet d'espérer une bonne dispersion des radio-sensibilisants. D'autre part, la densité élevée couplée au fort numéro atomique de l'élément permet d'espérer une bonne absorption des rayonnements.

Ces travaux ont été suivis d'études théoriques confirmant l'effet radio-sensibilisant de l'or dans le cadre d'un traitement aux rayons X et dans différentes situations courantes de traitements (S.H. Cho, Estimation of tumour dose enhancement due to gold nanoparticles during typical radiatio treatments : a preliminary Monte Carlo Study, Phys. Med. Biol. 50 (2005) N163).

Si on ne comprend pas encore précisément les mécanismes mis en jeu dans l'effet thérapeutique, l'utilisation de nanoparticules de métaux apparaît très intéressante. Elle reste cependant limitée à des métaux nobles, souvent précieux et très chers, car l'utilisation d'autres éléments sous forme métalliques risque d'entraîner des effets d'oxydation indésirables et difficilement contrôlables.

La demande de brevet française FR 2 877571 décrit des nanoparticules pourvues d'un élément de ciblage intracellulaire, leurs préparations et leurs utilisations. Les nanoparticules décrites dans ce document sont des nanoparticules composites pourvues d'un élément de ciblage intra-cellulaire capable de générer une réponse aux excitations électromagnétiques ou neutroniques. Ces nanoparticules comprennent un noyau comprenant au moins un composé inorganique et éventuellement un ou plusieurs autres composés organiques et peuvent être activées *in vivo* pour marquer ou altérer des cellules, des tissus ou des organes. Ce document cite de nombreux oxydes et hydroxydes inorganiques comme constituants du noyau mais n'envisage nullement l'utilisation spécifique des oxydes de lanthanides ni la possibilité, à condition de choisir cet élément comme constituant essentiel de la nanoparticule de ne pas avoir recours à une molécule de ciblage exposée à la surface de la nanoparticule.

On peut également citer le document WO2005/120590 qui décrit des particules activables utilisables dans le domaine de la santé, capables de générer des radicaux libres ou de la chaleur sous excitation aux rayons X. Les nanoparticules décrites dans ce document comportent un noyau comprenant un premier composé inorganique absorbant les rayons X et émettant de l'énergie UV visible et un second composé inorganique ou organique absorbant de l'énergie UV visible et produisant des radicaux libres au contact de l'eau ou de l'oxygène.

On a déjà décrit dans de nombreux documents de littérature des nanoparticules à base d'oxydes de lanthanides et en particulier à base d'oxydes de gadolinium. (par exemple : M. Engström, A. Klasson, H. Pedersen, C. Vahlberg, P.O. Käli, K. Uvdal, High proton relaxivity for gadolinium oxide nanoparticles, Magn. Reson. Mater. Phy (2006) 19, 180-186 et Hybrid gadolinium oxide nanoparticles : Multimodal contrast agents for in vivo imaging, J. Am. Chem. Soc. 2007, 129, 5076-5084 montrent comment ceux-ci peuvent être utilisés comme agents de contraste IRM).

On a en particulier décrit dans la demande internationale WO 2005/088314 (correspondant à FR 2 867 180) des nanoparticules hybrides comprenant un coeur constitué d'un oxyde de terres rares, éventuellement dopé avec une terre rare ou un actinide ou un mélange de terres rares ou bien un mélange de terres rares et d'actinides dans lequel au moins 50 % des ions métalliques sont des ions terres rares, un enrobage autour de ce coeur, constitué majoritairement de polysiloxanes fonctionnalisés et au moins un ligand biologique greffé par liaison covalente à l'enrobage polysiloxane. Les nanoparticules décrites dans ce document sont essentiellement utilisées pour des applications en tant que sondes pour la détection, le suivi et la quantification des systèmes biologiques.

Il a maintenant été découvert que des nanoparticules dont la structure peut, dans certains cas recouper celle des particules décrites dans la demande internationale WO 2005/088314, trouvent des applications particulièrement intéressantes en tant qu'agents radio-sensibilisants dans un traitement par radiothérapie au moyen de rayons X ou gamma. La présente invention concerne donc de nouveaux agents radio-sensibilisants particulièrement efficaces qui ne nécessitent pas le recours obligatoire à des biomolécules en surface pour cibler les cellules.

Par ailleurs, il est apparu aux inventeurs de la présente invention que l'utilisation de telles nanoparticules conduisait à des produits à la fois beaucoup plus économiques que ceux de l'art antérieur à base d'or et également beaucoup plus faciles à manipuler que ces produits métalliques qui sont souvent très réactifs et oxydables au sein du milieu biologique (s'il ne s'agit pas de métaux nobles) et plus difficile à fonctionnaliser de façon stable par l'intermédiaire de liaisons covalentes. De plus ces produits métalliques sont difficilement synthétisables en alliages d'éléments lourds et les combinaisons stables semblent très délicates à réaliser.

Plus précisément, l'invention propose de nouveaux agents radio-sensibilisants sous forme de nanoparticules dont la taille est comprise entre 1 et 50 nm, ces particules pouvant être soit constituées exclusivement d'un ou plusieurs oxydes ou oxohydroxydes de lanthanides ou être constitués, de façon particulièrement avantageuse, d'un coeur constitué d'oxydes ou d'oxohydroxydes de lanthanides et d'un enrobage soit inorganique, soit mixte organique-inorganique, constitué d'un polysiloxane, avec éventuellement des molécules organiques greffées en surface ou comprises en son sein.

L'utilisation de telles nanoparticules permet, en jouant sur la composition de ces nanoparticules d'obtenir simultanément un nombre important d'avantages supplémentaires qui ressortiront de l'ensemble de la description et des exemples et ce, avec des particules dont la fabrication s'avère particulièrement simple et économique, comparée à celle des particules à base de métaux précieux décrites dans l'art antérieur ou à celle nécessitant la présence de matériaux de ciblage.

Ainsi, selon une caractéristique essentielle de son premier objet, l'invention concerne l'utilisation de nanoparticules de dimensions comprises entre 1 et 50 nm, dont une partie au moins est constituée d'au moins un oxyde et/ou un oxohydroxyde d'au moins un lanthanide, lesdites nanoparticules étant :
- soit constituées d'au moins un oxyde et/ou un oxohydroxyde d'au moins un lanthanide,
- soit sous forme de nanoparticules comprenant un coeur constitué d'au moins un oxyde et/ou un oxohydroxyde d'au moins un lanthanide, et un enrobage constitué d'un polysiloxane, avec éventuellement des molécules organiques greffées en surface ou comprises en son sein,
en tant qu'agent radio-sensibilisant dans la fabrication d'une composition injectable destinée à améliorer l'efficacité du traitement d'une tumeur par irradiations X ou gamma.

Selon un deuxième objet, l'invention concerne, à titre de produits nouveaux, des nanoparticules s'avérant particulièrement intéressantes pour la mise en oeuvre de l'invention du fait qu'elles sont sous forme d'un coeur et d'un enrobage bien particulier.

Ces nanoparticules sont caractérisées en ce qu'elles sont constituées :
- d'un coeur constitué d'au moins un oxyde et/ou un oxohydroxyde d'au moins un lanthanide défini(s) de la même façon que dans le premier objet, ledit coeur présentant une dimension comprise entre 1 et 2 nm, et
- d'un enrobage dudit coeur en polysiloxane comprenant 1 à 5 silicium par lanthanide et dont au moins 10% des atomes de silicium dudit polysiloxane sont liés de façon covalente à des molécules organiques hydrophiles de masses molaires inférieures à 450 g/mol, de préférence choisies parmi les molécules organiques comportant des fonctions alcools ou acides carboxyliques ou amines ou amides ou esters ou éther-oxydes ou sulfonates ou phosphonates ou phosphinates.

L'invention concerne également des compositions injectables contenant les nanoparticules nouvelles de l'invention.

D'autres caractéristiques et avantages de l'invention apparaîtront dans la description qui suit ainsi que dans les exemples faisant référence aux figures 1 à 8 :
- la figure 1 donnée en référence à l'exemple 1 représente la mesure par spectroscopie de corrélation de photons de la taille et de la distribution en taille des nanoparticules,
- la figure 2 représente les images pondérées T2 du cerveau d'un rat porteur d'une tumeur implantée selon l'exemple 6,
- la figure 3 représente le pourcentage de survie de rats porteurs d'une tumeur cérébrale implantée selon l'exemple 6, après un traitement selon l'exemple 10,
- la figure 4 représente les images pondérées du cerveau d'un rat porteur d'une tumeur implantée selon l'exemple 6, après un traitement selon l'exemple 7,
- la figure 5 représente les images pondérées du cerveau d'un rat avant (figure 5a) et après (figure 5b) traitement selon l'exemple 9,
- la figure 6 représente le pourcentage de survie de rats porteurs d'une tumeur cérébrale implantée selon l'exemple 6, après un traitement selon l'exemple comparatif 12,
- la figure 7 représente le pourcentage de survie de rats porteurs d'une tumeur cérébrale implantée selon l'exemple 6, après un traitement selon l'exemple 14,
- la figure 8 représente le pourcentage de survie de rats porteurs d'une tumeur cérébrale implantée selon l'exemple 6, après un traitement selon l'exemple 15.

Les nanoparticules utilisées selon l'invention présentent des dimensions comprises entre 1 et 50 nm et peuvent être exclusivement constituées d'au moins un oxyde et/ou un oxohydroxyde d'au moins un lanthanide ou peuvent se présenter sous une forme enrobée.

Elles peuvent être exclusivement constituées d'un coeur comprenant au moins un oxyde ou oxohydroxyde d'au moins un lanthanide ou un mélange d'oxydes, un mélange d'oxohydroxydes ou un mélange d'oxyde et d'oxohydroxyde d'au moins un lanthanide ou peuvent se présenter sous la forme d'un coeur entouré d'un enrobage. Dans ce cas, le coeur comprend au moins un oxyde ou oxohydroxyde d'au moins un lanthanide ou un mélange d'oxydes, d'oxohydroxydes ou d'oxyde et d'oxohydroxyde d'au moins un lanthanide. Selon une variante de réalisation, les particules sont constituées exclusivement d'un oxyde de lanthanide, d'un oxyde mixte de lanthanides (c'est-à-dire d'au moins deux lanthanides), d'un oxohydroxyde de lanthanide, d'un oxohydroxyde mixte de lanthanides (c'est-à-dire d'au moins deux lanthanides) ou d'un de leur mélange. De même, dans le cas où les particules selon l'invention se trouvent sous une forme enrobée, selon une variante de réalisation, le coeur des particules est constitué exclusivement d'un oxyde de lanthanide, d'un oxyde mixte de lanthanides (c'est-à-dire d'au moins deux lanthanides), d'un oxohydroxyde de lanthanide, d'un oxohydroxyde mixte de lanthanides (c'est-à-dire d'au moins deux lanthanides) ou d'un de leur mélange.

L'enrobage, lorsqu'il est présent est constitué d'au moins un constituant inorganique ou mixte inorganique/organique.

Comme cela ressortira de la description et des exemples qui suivent, cet enrobage, lorsqu'il est présent, est avantageusement constitué d'un polysiloxane (qui constitue un constituant inorganique) mais peut également comprendre des molécules organiques soit directement fixées à la surface du coeur, soit greffées sur un enrobage inorganique, soit même comprises au sein de cet enrobage inorganique.

Les particules utilisées selon l'invention peuvent être uniquement, comme exposé précédemment, constituées d'oxydes et/ou d'oxohydroxydes de lanthanides.

Elles peuvent être également soit sous forme purement inorganique et comprendre dans ce cas un coeur constitué d'oxydes et/ou d'oxohydroxydes de lanthanides et enrobé d'un enrobage inorganique.

Elles peuvent également être sous forme de nanoparticules hybrides inorganiques et organiques et être constituées d'un coeur en oxydes et/ou oxohydroxydes d'au moins un lanthanide et un enrobage mixte organique/inorganique.

La taille hydrodynamique de la particule est mesurée en granulométrie laser par spectroscopie de corrélation de photons (Photon Corrélation Spectroscopy). Dans le cas où la particule est composite, la taille de chacune des couches successives est mesurée par cette technique après chaque étape de l'élaboration. La taille vraie de la particule et de ses différents constituants est mesurée par microscopie électronique à transmission. Les tailles données dans le présent document sont, sauf indications contraires, les tailles mesurées en microscopie électronique à transmission.

Les particules sont préférentiellement de forme coeur-coquille et de préférence sensiblement sphériques.

Plus généralement, le coeur d'oxyde ou d'oxohydroxyde de lanthanide peut être sphérique, facetté (les plans denses formant alors les interfaces) ou de forme allongée. De même alors, la particule finale est sphérique, facettée ou de forme allongée.

Le coeur ou la nanoparticule elle-même lorsqu'il n'y a pas d'enrobage fait avantageusement entre 1 et 30 nm.

Selon l'invention, on utilisera avantageusement des particules de faibles dimensions.

En effet, les particules de faibles dimensions présentent généralement une meilleure stabilité colloïdale et sont plus adaptées à des injections.

Leur faible taille et leur grande stabilité permet une meilleure diffusion :
∘ depuis le compartiment vasculaire vers les cellules cibles, par un passage de la barrière capillaire non limité (dû à leur petite taille) et à une diffusion élevée à travers les tissus interstitiels jusqu'aux cellules, après injection intravasculaire.
∘ La diffusion tissulaire de petites particules est élevée, responsable d'une rétention tissulaire plus importante après passage de la barrière capillaire.

Comme les tumeurs sont le plus souvent hypervascularisées (par rapport aux tissus sains), on obtient, même en l'absence de fonctionnalisation spécifique des particules, une captation tissulaire plus élevée au niveau des tumeurs comme attesté par les études IRM réalisées (voir exemples).

Comparativement aux molécules pour lesquelles l'élimination peut se faire aussi par les reins, les particules de faible taille ont un temps de résidence à l'intérieur de l'organisme plus long et permettent une accumulation préférentielle dans les zones d'intérêt (cf. exemple 8 montrant qu'il faut, en masse, 100 fois plus de lanthanides sous forme de complexes que sous forme nanoparticules, pour avoir le même effet).

La petite taille des particules permet une élimination urinaire des particules (contrairement à des particules plus grosses), permettant l'élimination rapide de la fraction des particules non captées au niveau des tissus tumoraux. Elle limite la captation non spécifique par les cellules du système réticulo-endothélial. Cette biodistribution très favorable a été confirmée par les études IRM.

Après absorption d'un rayon X ou gamma, les lanthanides peuvent émettre des électrons secondaires (d'une énergie de quelques keV), Auger ou Compton (d'une énergie de quelques keV pour des rayons X ayant une énergie inférieure à 100 keV). La faible taille de la particule permet l'échappement des électrons même si le lanthanide excité se trouve au centre de la particule [les électrons Auger traversant avec une absorption négligeable une épaisseur de quelques nanomètres et les électrons secondaires et Compton une épaisseur d'une dizaine de nanomètres].

La nanoparticule de faible taille permet ainsi :
- un temps de résidence long et une perméation accrue dans les zones lésées (contrairement aux molécules)
- une élimination rénale facilitée (par rapport aux particules de taille supérieure)
- une efficacité de traitement ionisant accrue (par rapport aux particules de taille supérieure)
- une moindre sensibilité aux systèmes d'éliminations classiques par exemple le foie par les opsonisations (par rapport aux particules de taille supérieure)

Pour toutes les raisons exposées ci-dessus et, comme le montrent les résultats présentés dans les exemples, on utilise selon la présente invention de préférence des nanoparticules ayant des dimensions inférieures à 5 nm et de préférence inférieures à 2 nm, lorsqu'il s'agit de particules exclusivement constituées d'au moins un oxyde et/ou d'un oxohydroxyde d'au moins un lanthanide ou présentant un coeur de dimensions inférieures à 5 nm, et de préférence inférieures à 2 nm, lorsqu'il s'agit d'une particule sous forme de coeur et d'enrobage.

Selon une variante particulièrement avantageuse de l'invention, la partie de la nanoparticule contenant les oxydes et/ou les oxohydroxydes comprendra au moins deux lanthanides différents, représentant chacun plus de 10 % en masse de la totalité des lanthanides et, de préférence, plus de 20 %.

Cette variante présente un avantage tout particulier puisqu'elle permet d'adapter la particule à la source de rayons X ou gamma.

En effet, de nombreuses sources d'irradiation ne sont pas monochromatiques mais polychromatiques (source compacte, rayonnement synchrotron poly- ou monochromatique). Il peut donc être intéressant d'adapter la composition en lanthanides à la dispersion en énergie du rayonnement, de manière à absorber l'ensemble du spectre et non pas seulement une énergie particulière.

Le choix de la nature et de la proportion des lanthanides (dont l'énergie de seuil varie de 39 keV pour le lanthane à 63 keV pour le lutétium) se fera pour absorber les maxima d'intensité du rayonnement.

Dans un tel cas où l'on utilisera plusieurs lanthanides différents, les oxydes et/ou oxohydroxydes de ces différents lanthanides pourront être soit en couches successives, soit sous forme d'une solution solide.

La solution solide est plus facile à réaliser du point de vue de la synthèse. Par ailleurs, une structure multicouches peut être intéressante si on veut augmenter le contraste en imagerie par résonance magnétique (en mettant les lanthanides à fort contraste en surface) ou si pour une meilleure efficacité du traitement on dispose en surface les lanthanides émettant les électrons de plus faible énergie

Le choix des lanthanides qui ont tous des chimies très proches permet de réaliser aisément des solutions solides adaptables.

Un des avantages de l'utilisation des nanoparticules de l'invention est que, comme cela est bien connu, un certain nombre de lanthanides donnent des signaux en imagerie par résonance magnétique (IRM), ce qui permet, à condition de bien choisir les proportions et la nature des lanthanides de provoquer pendant la thérapie un signal en IRM, permettant une détection *in vivo* de la présence des particules et/ou un monitorage de la thérapie.

Ainsi, les lanthanides contiendront avantageusement au moins 50 % en masse de gadolinium (Gd), de dysprosium (Dy), d'holmium (Ho) ou de mélanges de ces lanthanides.

La combinaison d'un effet thérapeutique et de propriétés de détectabilité par imagerie *in vivo* est un atout majeur des agents thérapeutiques.

En effet, l'imagerie *in vivo* va permettre de quantifier la captation tissulaire tumorale des nanoparticules, d'établir la cinétique de captation pour connaître le délai après injection où celle-ci est maximale dans la tumeur. Cette connaissance permet de déterminer de manière optimale le délai dans lequel doit être réalisée l'irradiation externe, pour obtenir un effet maximal (effet radio-sensibilisant maximal). La détection externe par imagerie de ces nanoparticules permet donc de monitorer la thérapie.

Ces nanoparticules sont responsables d'une modification positive du contraste IRM qui peut être quantifiée. L'imagerie par Résonance Magnétique permet d'obtenir des images de haute résolution sans effet iatrogène.

Selon une variante de l'invention particulièrement intéressante lorsque l'on couple le procédé de radiothérapie avec un suivi par IRM, on utilise des nanoparticules dans lesquelles la partie contenant des oxydes et/ou oxohydroxydes de lanthanides contient à sa périphérie des lanthanides provoquant un signal IRM, de préférence du gadolinium, et au moins un autre lanthanide dans sa partie centrale.

En effet, le contraste en IRM se faisant par une action de l'ion paramagnétique dans le périmètre de ses premières couches de coordination, il est préférable de mettre les lanthanides qui agiront comme agents de contraste en surface du coeur.

Les lanthanides de fort numéro atomique absorbant les rayonnements seront alors préférentiellement situés au centre de ce coeur. Pour que les électrons Auger générés par l'absorption du rayonnement puissent s'extraire de la particule (distance d'échappement de l'ordre du nanomètre), il est alors souhaitable que celle-ci soit de faible taille.

Enfin, selon une autre variante avantageuse de l'invention liée au fait qu'il est connu qu'un certain nombre de lanthanides présente des sections efficaces de capture permettant leur utilisation dans des traitements par neutron-thérapie, on pourra choisir, si besoin est dans la thérapie envisagée, un lanthanide présentant une section efficace de capture suffisante à cet effet, de façon à permettre également un traitement par neutron-thérapie.

A cet effet, le choix du gadolinium s'avère particulièrement intéressant et on utilisera avantageusement, surtout si l'on veut coupler le traitement de radiothérapie avec un traitement par neutron-thérapie des lanthanides constitués d'au moins 50 % en masse de gadolinium.

Enfin, on utilisera avantageusement des oxydes et/ou des hydroxydes de lanthanides comprenant au moins 30 % en masse d'oxydes de lutétium ou d'ytterbium ou d'un de leurs mélanges.

On augmentera ainsi la probabilité d'interaction qui est directement reliée à la densité et au numéro atomique élevé des éléments en interaction. L'utilisation de lutétium, le lanthanide au numéro atomique le plus élevé et dont l'oxyde est le plus dense, est ainsi particulièrement avantageuse.

Comme exposé précédemment, les nanoparticules utilisées selon l'invention sont avantageusement sous la forme d'un coeur et d'un enrobage.

Cet enrobage peut être soit inorganique, soit organique et inorganique.

Selon une variante de l'invention, on choisira un enrobage constitué de polysiloxane, sur lequel on fixe les molécules, de façon particulièrement avantageuse, des molécules organiques hydrophiles.

Comme exposé précédemment, l'enrobage peut être un enrobage inorganique ou mixte organique. D'une façon générale dans le cas où les nanoparticules présentent un enrobage, il est avantageux que les oxydes et/ou oxohydroxydes de lanthanides représentent au moins 30 % en masse par rapport à l'ensemble des constituants inorganiques de ladite nanoparticule, les constituants organiques étant liés de façon covalente à la nanoparticule hybride et représentant moins de 30 % en masse de la particule finale.

On notera que lorsque l'on parle de "l'ensemble des constituants inorganiques", on compte dans cet ensemble les oxydes et oxohydroxydes de lanthanides.

D'une façon générale, la fonction de l'enrobage est multiple.

Il s'agit d'une part de protéger le coeur d'une dissolution inopportune et d'autre part d'adapter facilement la stabilité des nanoparticules au système d'injection (éviter des agglomérations incontrôlés) du fait des surfactants nombreux qu'il est possible de fixer sur sa surface.

Enfin, une autre fonction de l'enrobage est d'adapter les charges et la chimie de surface pour améliorer la biodistribution et favoriser les surconcentrations locales dans les zones tumorales.

A cet effet, le polysiloxane, enrobage choisi dans le cadre de l'invention est particulièrement avantageux. L'enrobage peut être constitué exclusivement de polysiloxane ou exclusivement de polysiloxane et de molécules organiques.

Selon une variante particulièrement avantageuse, les nanoparticules utilisées selon la présente invention sont sous forme enrobée et l'enrobage est constitué de polysiloxane qui représente de 1 à 70 % en masse des constituants inorganiques de la nanoparticule.

Cet enrobage sous forme de polysiloxane est avantageusement tel que le nombre d'atomes de silicium par rapport au nombre d'atomes de lanthanides est compris entre 0,1 et 8.

Le choix de ce rapport permet de protéger de façon efficace le coeur de la nanoparticule et/ou d'adapter sa stabilisation colloïdale et/ou d'adapter son interaction magnétique avec l'eau et par conséquent ses caractéristiques d'agents de contraste.

Comme expliqué précédemment, l'enrobage est avantageusement un enrobage en polysiloxane et comprend en outre des molécules organiques hydrophiles de masses molaires inférieures à 5 000 g/mol, de préférence inférieures à 800 g/mol, de préférence encore inférieures à 450 g/mol.

Ces molécules organiques comprennent avantageusement les motifs suivants donnés ci-dessous avec leur masse molaire (Mw) préférée :
- poly(éthylèneglycol) bis(carboxyméthyl) éther (PEG), 250< Mw<5000 g.mol⁻¹
- Polyoxyéthylène bis(amine), 250< Mw<5000 g.mol⁻¹
- O-Méthyl-O'-succinylpolyéthylèneglycol, Mw de l'ordre de 2000 g.mol⁻¹
- Méthoxypolyéthylèneglycolamine (Mw de l'ordre de 750 g.mol⁻¹
- Acide diéthylènetriaminepentaacétique (DTPA) et ses dérivés, en particulier le DTPA dithiolé (DTDTPA)
- 1,4,7,10-Tétraazacyclododécane-1,4,7,10- acide tétraacétique (DOTA) et dérivés
- Acide succinique et mercaptosuccinique
- Glucose et dérivés, par exemple le dextran Acides aminés ou peptides hydrophiles (acide aspartique, acide glutamique, lysine, cystéine, sérine, thréonine, glycine...)

Plus généralement, les molécules organiques comprendront avantageusement des fonctions alcools ou acides carboxyliques ou amines ou amides ou esters ou éther-oxydes ou sulfonates ou phosphonates ou phosphinates et seront liées de préférence de façon covalente, à au moins 10 % des atomes de silicium dudit polysiloxane.

On choisira de préférence les molécules organiques comprenant un motif polyéthylèneglycol, DTPA, DTDTPA (DTPA dithiolé) ou un acide succinique.

Parmi les molécules organiques ci-dessus, on choisira avantageusement celles qui présentent des propriétés complexantes des lanthanides, en particulier celles dont la constante de complexation est supérieure à 10¹⁵.

A cet effet, on choisira de préférence le DTPA ou le DOTA.

On comprendra aisément que, en fonction de l'application visée, en particulier en fonction de la nature de la tumeur à traiter, de la technique d'injection et de la dose de rayonnement, la proportion d'oxydes et/ou d'oxohydroxydes de lanthanides peut varier dans de larges proportions. Toutefois, d'une façon générale, les compositions utilisées selon l'invention contiendront avantageusement entre 0,5 et 200 g/L d'oxyde et/ou d'oxohydroxydes de lanthanides.

La composition sera injectée dans l'organisme soit directement dans la tumeur à traiter, soit par voie parentérale, en particulier intraveineuse.

L'irradiation, par les techniques classiques de radiothérapies ou curiethérapie, sera alors réalisée soit directement après injection soit après un temps déterminé pour avoir le maximum d'efficacité et de sélectivité entre tissu sain et tissu marqué. Ce temps d'attente et la dose d'irradiation pourront avantageusement être déterminés en observant le positionnement et le devenir des nanoparticules après injection, par exemple par IRM.

Parmi les nanoparticules décrites ci-dessus et pouvant être avantageusement utilisées dans le cadre de l'invention, comme exposé précédemment, un certain nombre sont nouvelles et constituent des produits nouveaux en eux-mêmes.

Il s'agit de nanoparticules sous forme enrobée et comprenant un coeur dont la dimension est inférieure à 2 nm et, de préférence, comprise entre 1 et 2 nm.

Ces nanoparticules comprennent un enrobage constitué de polysiloxane, la nanoparticule comprenant 1 à 5 silicium par lanthanide et, au moins 10 % des atomes de silicium sont liés à des molécules organiques hydrophiles de masses molaires inférieures à 450 g/mol, de préférence choisies parmi les molécules organiques comportant des fonctions alcools ou acides carboxyliques ou amines ou amides ou esters ou éther-oxydes ou sulfonates ou phosphonates ou phosphinates, liées de façon covalente à au moins 10 % des atomes de silicium dudit polysiloxane.

Selon une variante avantageuse, lesdites molécules organiques hydrophiles contiennent un motif polyéthylèneglycol, DTPA, DTDTPA (DTPA dithiolé) ou un acide succinique.

Selon une autre variante avantageuse, lesdites molécules organiques hydrophiles sont des complexants des lanthanides dont la constante de complexation est supérieure à 10¹⁵, de préférence le DTPA ou le DOTA.

Ces molécules organiques seront avantageusement choisies parmi celles présentant un ou plusieurs acides carboxyliques par exemple le DTPA et ses dérivés.

On notera que tous ces produits nouveaux font partie de ceux dont l'utilisation est préférée dans le cadre de l'invention.

En effet, même si toutes les nanoparticules utilisables selon l'invention s'avèrent particulièrement utiles en tant qu'agents radio-sensibilisants, notamment du fait de la présence des oxydes et/ou oxohydroxydes de lanthanides qui interagissent efficacement avec les rayonnements énergétiques X ou gamma, les particules enrobées présentant un coeur de dimensions très réduites et comprenant un enrobage en polysiloxane, et de préférence un enrobage en polysiloxane sur lequel sont greffées des molécules organiques telles que définies précédemment s'avèrent particulièrement efficaces pour augmenter l'efficacité thérapeutique d'un traitement par rayons X ou gamma, comme cela ressort des exemples ci-après.

Les nanoparticules hybrides et tout particulièrement celles dont le coeur est particulièrement fin, de préférence de dimensions inférieures à 5 nm et de préférence encore comprises entre 1 et 2 nm permettent d'obtenir une amplification de l'effet dose très importante, même supérieure à ce qui est observé avec des nanoparticules d'or, comme cela ressort des exemples ci-après.

La présence d'une couche de polysiloxane confère, après injection *in vivo,* à la nanoparticule un comportement en adéquation avec l'objectif d'irradiation recherché.

Par ailleurs, la très faible taille du coeur et du revêtement permet, en fonction de la fonctionnalisation, d'obtenir des particules suffisamment stables et contrôlables pour des injections *in vivo,* intraveineuses ou directement intratumorales.

D'une façon générale, les nanoparticules utilisées selon l'invention, et plus particulièrement les nanoparticules nouvelles, permettent d'obtenir localement au niveau de la tumeur une surconcentration. Ces particules peuvent être conçues pour présenter localement au niveau de la tumeur une surconcentration (soit directement reliée à l'augmentation de la zone d'irradiation sanguine, soit liée à une diffusion passive au sein de la tumeur, soit par fonctionnalisation avec des biomolécules actives).

Un grand intérêt de la méthode vient de l'utilisation préférentielle de nanoparticules de lanthanides oxydés présentant des propriétés magnétiques intéressantes et pouvant être suivies par imagerie IRM directement.

Cette technique permet alors d'optimiser le moment entre les injections et les thérapies. Elle permet également de localiser au mieux les zones à irradier.

Un autre grand intérêt de la méthode consiste à utiliser des coeurs à base de lanthanides oxydés mixtes, c'est-à-dire contenant plusieurs lanthanides différents, ou utilisant un mélange de nanoparticules hybrides à coeur différents.

Par cette approche, on peut ainsi adapter au mieux le radio-sensibilisant au spectre énergétique de la source d'irradiation, à la thérapie et au patient.

D'une façon générale, l'homme du métier pourra aisément fabriquer des nanoparticules utilisées selon l'invention et, en particulier les particules nouvelles, en s'appuyant sur différents documents de littérature.

Plus précisément, en ce qui concerne la synthèse des différentes nanoparticules utilisées selon l'invention, on notera les éléments suivants :
- les oxydes et oxohydroxydes de lanthanides sont préférentiellement fabriqués selon des procédés utilisant un polyol comme solvant, par exemple selon l'approche décrite dans la publication de R. Bazzi et al. (Bazzi, R.; Flores, M. A.; Louis, C.; Lebbou, K.; Zhang, W.; Dujardin, C.; Roux, S.; Mercier, B.; Ledoux, G.; Bernstein, E.; Perriat, P.; Tillement, O. Synthesis and properties of europium-based phosphors on the nanometer scale: Eu2O3, Gd2O3:Eu, and Y2O3:Eu. Journal of Colloid and Interface Science (2004), 273(1), 191-197.) ou dans la publication M. Flores et al. (Flores-Gonzalez, M. ; Ledoux, G. ; Roux, S. ; Lebbou, K. ; Perriat, P. ; Tillement, O. ; Preparing nanometer sacled Tb-doped Y2O3 luminescent powders by the polyol method. Journal of Solid State Chemistry (2005),178, 989-997.). Ils peuvent également être réalisés par synthèse lyophilisation et traitement thermique partiel de décomposition, comme décrit dans la publication de C. Louis et al. (Louis, C.; Bazzi, R.; Flores, M.o A.; Zheng, W.; Lebbou, K.; Tillement, O.; Mercier, B.; Dujardin, C.; Perriat, P. Synthesis and characterization of Gd2O3:Eu3+ phosphor nanoparticles by a sol-lyophilization technique. Journal of Solid State Chemistry (2003), 173(2), 335-341.).
- Pour l'enrobage avec des couches de polysiloxanes, plusieurs techniques peuvent être employées, dérivées de celles initiées par Stoeber (Stober, W; J Colloid Interf Sci 1968, 26, 62). On peut également utiliser le procédé employé pour l'enrobage de particules comme présenté dans la publication de C. Louis et al. (Louis, C. ; Bazzi, R. ; Marquette, C. ; Bridot, J. L. ; Roux, S ; Ledoux, G. ; Mercier, B. ; Blum, L. ; Perriat, P. ; Tillement, O. ; Nanosized hybrid particles with double luminescence for biological labeling, Chemistry of Marerials (2005), 17, 1673-1682.) et la demande internationale WO 2005/088314.
- Pour le greffage organique de surface, il est conseillé d'utiliser directement des silanes contenant des fonctions organiques hydrophiles ou passer en deux étapes en greffant sur les composés de surface aminés par exemple en cas d'utilisation d'APTES.
- La fonctionnalisation en surface des nanoparticules d'oxyde de lanthanide enrobées vise, entre autres, à introduire des molécules hydrophiles afin de favoriser la libre circulation des nanoparticules dans le système vasculaire et d'éviter ainsi une accumulation non-spécifique indésirable. Une des stratégies possibles consiste à insérer dans la couche de polysiloxane (lors de sa formation) des organoalcoxysilanes dont la partie organique est hydrophile par exemple des chaînes de PEG terminées par un groupement alcoxysilyle, un dérivé du glucose ou un groupement phosphonate lié à un groupement alcoxysilyle. Une autre voie permettant de rendre les particules parfaitement circulantes consiste à post-fonctionnaliser la couche de polysiloxane par des molécules hydrophiles possédant un groupe réactif capable d'assurer l'accrochage de la molécule sur la couche de polysiloxane qui doit posséder des sites de greffage. Il est par conséquent nécessaire d'utiliser pour confectionner cette couche de polysiloxane des organoalcoxysilanes possédant des groupements réactifs tels que les dérivés de l'anhydride succinique, des amines, des thiols, des maléimides, des acides carboxyliques, isocyanate, isothiocyanate, époxyde sur lesquels viendront s'accrocher par liaison covalente (à la suite d'une réaction de condensation) les molécules hydrophiles. Pour la plupart les entités hydrophiles comportent une chaine hydrocarbonée avec au moins une fonction ionisable (acide carboxylique, amine, phosphate, phosphinate, phosphonate, sulfonate) et/ou plusieurs fonctions alcool et/ou éther-oxyde et/ou thiol.

En ce qui concerne le traitement par radiothérapie, l'homme du métier comprendra aisément que la source sera adaptée à la nature de la nanoparticule ainsi qu'au type de tumeur à traiter.

Ainsi, l'utilisation du gadolinium dans un contexte thérapeutique nécessite l'utilisation d'une source de rayons X présentant une énergie critique située au dessus du seuil d'absorption K du gadolinium (53,4 keV). Cette source peut-etre monochromatique afin d'être sélectif pour l'élément considéré, ici le gadodinium (technique de SSRT de l'ESRF) et cela semble être la meilleure configuration. Elle peut être aussi polychromatique (faisceau blanc de l'ESRF) et être utilisée en mode MRT (fractionnement du faisceau en microfaisceaux et dépôt de dose de l'ordre de 600Gy). De plus, de façon plus conventionnelle, le gadolinium doit être aussi intéressant si utilisé avec un irradiateur conventionnel de spectre adapté.

### Exemples

### Exemple 1. Synthèse de coeur d'oxyde de gadolinium

Un colloïde est préparé par dissolution d'une quantité de 56 g.L⁻¹ de sels de chlorures de gadolinium dans un volume de 1L de diéthylène glycol. A la solution obtenue, un ajout de 45mL de soude à une concentration de 3M est effectué à température ambiante en 1h30. Le mélange est ensuite chauffé à 180°C pendant 4h. La taille finale des particules, mesurée par granulométrie, est d'environ 1,5nm.

Autour de ces particules, une couche de polysiloxane fonctionnalisé d'une épaisseur de 0,5nm est synthétisée par voie sol-gel.

Dans une solution contenant 200mL de colloïde et 800mL de diéthylène glycol, on ajoute 3,153mL d'aminopropyltriéthoxysilane (APTES), 2,008mL de tétraéthylorthosilicate (TEOS) et 7,650mL d'une solution aqueuse de 0,1M de triéthylamine.

La réaction s'effectue à 40°C dans un bain d'huile et sous agitation en plusieurs étapes.
à t = 0h ajout de 3% du volume d'APTES et de TEOS
à t = 1h ajout de 3% du volume d'eau total
à t = 2h ajout de 7% du volume d'APTES et de TEOS
à t = 3h ajout de 7% du volume d'eau total
à t = 4h ajout de 10% du volume d'APTES et de TEOS
à t = 5h ajout de 10% du volume d'eau total
à t = 6h ajout de 15% du volume d'APTES et de TEOS
à t = 7h ajout de 15% du volume d'eau total
à t = 23h ajout de 15% du volume d'APTES et de TEOS
à t = 24h ajout de 15% du volume d'eau total
à t = 25h ajout de 25% du volume d'APTES et de TEOS
à t = 26h ajout de 25% du volume d'eau total
à t = 27h ajout des 25% restant du volume d'APTES et de TEOS
à t = 28h ajout des 25% restant du volume d'eau total
à t = 76h fin de la synthèse

Les particules ainsi enrobées sont ensuite fonctionnalisées :
- Soit par du poly(éthylène glycol) bis(carboxyméthyl) éther (Mₙ = 250) (PEG250) :
   0,3612g de N-(3-Dimethylaminopropyl)-N'-ethylcarbodiimide, 0,3489g de pentafluorophénol et 181,5 µL de PEG250 sont dissous dans 5mL d'isopropanol sous agitation pendant 1h30. La solution obtenue est alors mélangée à 100mL de solution de nanoparticules enrobées. Le mélange est ensuite agité pendant 15h.

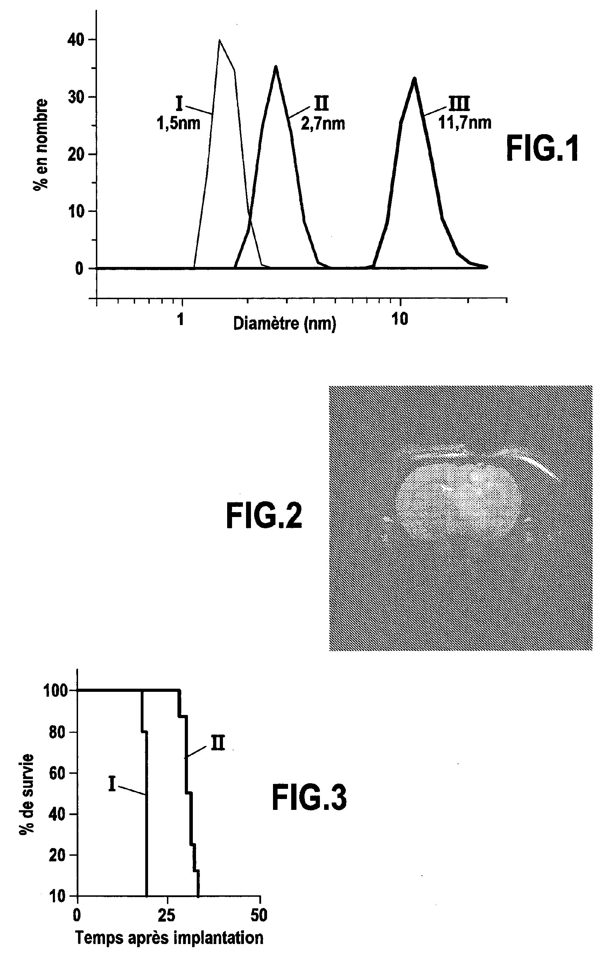

La figure 1 donne la mesure par spectroscopie de corrélation de photons (PCS, avec un analyseur en taille Zetasizer NanoS Malvern Instrument) de la taille et de la distribution en taille des nanoparticules préparées selon cet exemple, et plus précisément :
- Courbe 1 : nanoparticules d'oxyde de gadolinium avant enrobage ;
- Courbe II : nanoparticules d'oxyde de gadolinium après enrobage par une couche de polysiloxane ;
- Courbe III : après greffage de PEG250 sur les sites de greffage (amine de l'APTES) de la couche de polysiloxane.
- Soit par de l'acide diéthylènetriaminepentaacétique (DTPA) :
   0,616g de DTPA sont dissous dans 17,3mL de diméthylsulfoxide anhydre (DMSO) sous agitation pendant 1h30. La solution obtenue est alors mélangée à 100mL de solution de nanoparticules enrobées. Le mélange est ensuite agité pendant 15h.

### Exemple 2. Synthèse de coeur d'oxyde d'holmium

Un colloïde est préparé par dissolution d'une quantité de 56 g.L⁻¹ de sels de chlorures d'holmium dans un volume de 200mL de diéthylène glycol. A la solution obtenue, un ajout de 7,5mL de soude à une concentration de 3M est effectué à température ambiante en 1h30. Le mélange est ensuite chauffé à 180°C pendant 4h. La taille finale des particules, mesurée par granulométrie est d'environ 1,5nm.

Autour de ces particules, une couche de polysiloxane fonctionnalisé d'une épaisseur de 0,5nm est synthétisée par voie sol-gel.

Dans une solution contenant 200mL de colloïde et 800mL de diéthylène glycol, on ajoute 3,153mL d'aminopropyltriéthoxysilane (APTES), 2,008mL de tétraéthylorthosilicate (TEOS) et 7,650mL d'une solution aqueuse de 0,1M de triéthylamine.

La réaction s'effectue à 40°C dans un bain d'huile et sous agitation en plusieurs étapes.
à t = 0h ajout de 3% du volume d'APTES et de TEOS
à t = 1h ajout de 3% du volume d'eau total
à t = 2h ajout de 7% du volume d'APTES et de TEOS
à t = 3h ajout de 7% du volume d'eau total
à t = 4h ajout de 10% du volume d'APTES et de TEOS
à t = 5h ajout de 10% du volume d'eau total
à t = 6h ajout de 15% du volume d'APTES et de TEOS
à t = 7h ajout de 15% du volume d'eau total
à t = 23h ajout de 15% du volume d'APTES et de TEOS
à t = 24h ajout de 15% du volume d'eau total
à t = 25h ajout de 25% du volume d'APTES et de TEOS
à t = 26h ajout de 25% du volume d'eau total
à t = 27h ajout des 25% restant du volume d'APTES et de TEOS
à t = 28h ajout des 25% restant du volume d'eau total
à t = 76h fin de la synthèse

Les particules ainsi enrobées sont ensuite fonctionnalisées :
- Soit par du poly(éthylène glycol) bis(carboxyméthyl) éther (Mₙ = 250) (PEG250) :
   0,3612g de N-(3-Dimethylaminopropyl)-N'-ethylcarbodiimide, 0,3489g de pentafluorophénol et 181,5 µL de PEG250 sont dissous dans 5mL d'isopropanol sous agitation pendant 1h30. La solution obtenue est alors mélangée à 100mL de solution de nanoparticules enrobées. Le mélange est ensuite agité pendant 15h.
- Soit par de l'acide diéthylènetriaminepentaacétique (DTPA) :
   0,616g de DTPA sont dissous dans 17,3mL de diméthylsulfoxide anhydre (DMSO) sous agitation pendant 1h30. La solution obtenue est alors mélangée à 100mL de solution de nanoparticules enrobées. Le mélange est ensuite agité pendant 15h.

### Exemple 3. Synthèse d'oxyde mixte Gd/Ho (50/50)

Un colloïde est préparé par dissolution d'une quantité de 56 g.L⁻¹ de sels de chlorures d'holmium et de gadolinium dans un volume de 200mL de diéthylène glycol. A la solution obtenue, un ajout de 7,5mL de soude à une concentration de 3M est effectué à température ambiante en 1h30. Le mélange est ensuite chauffé à 180°C pendant 4h. La taille finale des particules, mesurée par granulométrie est d'environ 1,3nm.

Dans une solution contenant 40mL de colloïde et 160mL de diéthylène glycol, on ajoute 0,421mL d'aminopropyltriéthoxysilane (APTES), 0,267mL de tétraéthylorthosilicate (TEOS) et 1,020mL d'une solution aqueuse de 0,1M de triéthylamine.

La réaction s'effectue à 40°C dans un bain d'huile et sous agitation en plusieurs étapes.
à t = 0h ajout de 10% du volume d'APTES et de TEOS
à t = 1h ajout de 10% du volume d'eau total
à t = 2h ajout de 25% du volume d'APTES et de TEOS
à t = 3h ajout de 25% du volume d'eau total
à t = 4h ajout des 65% restant du volume d'APTES et de TEOS
à t = 5h ajout des 65% restant du volume d'eau total
à t = 53h fin de la synthèse.

### Exemple 4. Synthèse de nanoparticules d'oxyde d'environ 50 nm

Un colloïde est préparé par dissolution d'une quantité de 56 g.L⁻¹ de sels de chlorure de gadolinium dans un volume de 200mL de diéthylène glycol. A la solution obtenue, un ajout de 30 mL de soude à une concentration de 3M est effectué à température ambiante en 2h.

La taille des particules obtenues, mesurée par granulométrie est d'environ 50nm.

### Exemple 5. Formulation des solutions injectables

Les particules d'oxyde de lanthanide des exemples 1 à 3 sont purifiées par une succession de dialyses contre un mélange de diéthylène glycol et d'éthanol avec une proportion croissante d'éthanol (jusqu'à 100%) et dont le volume est environ vingt fois supérieur à celui de la solution à purifier. La purification est validée par une analyse élémentaire par ICP-MS.

Après purification, la solution colloïdale est reconcentrée dans du PEG400 (poly(éthylène glycol) de masse moléculaire 400 g.mol⁻¹) par ajout d'un volume défini de PEG400 et élimination sous pression réduite de l'éthanol. Les particules ainsi conservées peuvent être stockées plusieurs mois à forte concentration (jusqu'à 200 mM).

Les solutions injectables sont préparées en diluant les solutions de PEG400 à forte concentration de nanoparticules avec une solution d'HEPES et de NaCl pour obtenir au final une concentration en HEPES et en NaCl de 10 et 145 mM, respectivement. La concentration en nanoparticules est telle que [Gd] est comprise entre 0,1 et 25 mM.

Par exemple, à 0,2 mL d'une solution de nanoparticules d'oxyde de gadolinium préparées selon l'exemple 1 et concentrées dans le PEG400 ([Gd] = 85 mM) sont ajoutés par portion 3,2 mL d'une solution aqueuse contenant 10,6 mM d'HEPES et 154 mM de NaCl. Après chaque ajout la solution est vigoureusement agitée. Avant injection la solution est filtrée à travers des membranes dont la taille des pores est de 220 nm.

### Exemple 6. Implantation des tumeurs cérébrales à des rats, imagerie et survie

L'implantation des tumeurs cérébrales (gliosarcomes) est effectuée quinze jours avant le traitement par injection stéréotaxique de cellules 9L (10⁴ cellules dans 1µL) au niveau du noyau caudé droit de rats Fisher de 300 g (coordonnées : 3,5-5,5). Un examen d'imagerie par résonance magnétique (IRM) pratiqué quinze jours après l'implantation avec un imageur 7T (Biospec System 70/20, Brucker, Ettlingen, Germany) révèle, sur les images pondérées T₂ (TE = 76,3 ms, TR = 4488 ms, épaisseur de coupes = 0,6 mm, résolution spatiale = 0,0117 * 0,0156 cm/pixel), la présence de la tumeur qui n'est cependant pas visible sur les images pondérées T₁ (TE = 5,6 ms, TR = 304,6 ms, épaisseur de coupes = 0,6 mm, résolution spatiale = 0,0088 * 0,0130 cm/pixel) (cf. figure 2 qui présente les images pondérées T2 du cerveau d'un rat porteur d'une tumeur implantée selon cet exemple). La zone gris clair dans la partie droite du cerveau délimite l'emplacement de la tumeur.

Sans traitement, c'est-à-dire sans qu'aucune particule ne leur soit administrée et sans qu'aucune dose n'ait été délivrée, les rats porteurs de ce genre de tumeurs décèdent en moyenne au bout de 19 jours.

### Exemple 7. IRM de cerveau de rats après injection par voie intratumorale de nanoparticules d'oxyde de gadolinium

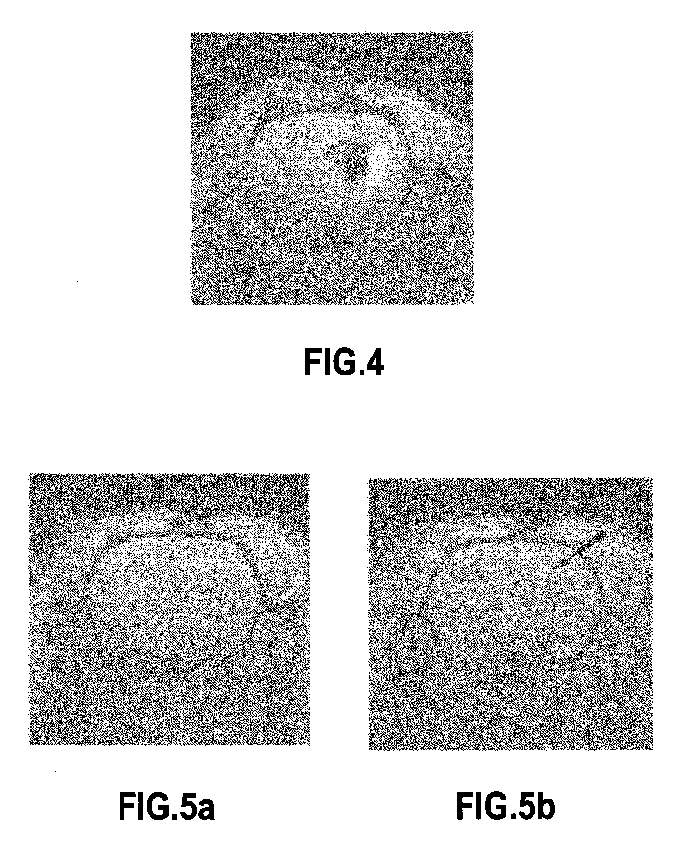

15 µL d'une solution injectable préparée selon l'exemple 5 ([Gd] = 5 mM) ont été injectés directement au centre de la tumeur (c'est-à-dire en réutilisant le site de trépanation utilisé au moment de l'injection des cellules) à une vitesse de 30 µL/h. Alors qu'il a été démontré que les nanoparticules d'oxyde de gadolinium génèrent un contraste positif qui se traduit par un éclaircissement de la zone où se sont accumulées les nanoparticules, l'examen des images pondérées T₁ (TE = 5,6 ms, TR = 304,6 ms, épaisseur de coupes = 0,6 mm, résolution spatiale = 0,0088 * 0,0130 cm/pixel) obtenues après l'injection (entre 2 et 40 minutes après l'injection) révèle un assombrissement de l'image dans la zone tumorale avec un blanchiment à la périphérie. Cet assombrissement résulte d'une forte concentration en élément paramagnétique : les particules se sont fortement concentrées dans la zone tumorale avec une diminution de la concentration du centre vers la périphérie. L'injection intratumorale de ces particules facilite donc la visualisation de la tumeur sur des images T1 alors qu'elle est invisible en l'absence de nanoparticules (cf. figure 4 qui montre les images pondérées T₁ du cerveau d'un rat après injection intratumorale d'une solution injectable de nanoparticules d'oxyde de gadolinium (selon cet exemple).

### Exemple 8 (comparatif). IRM de cerveau de rats après injection par voie intratumorale de complexes de gadolinium

15 µL d'une solution commerciale de complexes de gadolinium ([Gd] = 500 mM) ont été injectés directement au centre de la tumeur (c'est-à-dire en réutilisant le site de trépanation utilisé au moment de l'injection des cellules) à une vitesse de 30 µL/h. Cette solution est très largement utilisée pour les examens cliniques. La lecture des images pondérées T₁ (TE = 5,6 ms, TR = 304,6 ms, épaisseur de coupes = 0,6 mm, résolution spatiale = 0,0088 * 0,0130 cm/pixel) obtenues après l'injection (entre 2 et 40 minutes après l'injection) révèle un assombrissement de l'image dans la zone tumorale avec un blanchiment à la périphérie. Cet assombrissement résulte d'une forte concentration en élément paramagnétique : les molécules se sont fortement concentrées dans la zone tumorale avec une diminution de la concentration du centre vers la périphérie. L'injection intratumorale de ces molécules facilite donc la visualisation de la tumeur sur des images T₁ alors qu'elle est invisible en l'absence de nanoparticules. Il faut toutefois noter que la concentration en gadolinium est 100 fois supérieure à celle des solutions colloïdales de nanoparticules hybrides d'oxyde de gadolinium qui ont été injectées (exemple 7).

### Exemple 9. IRM de cerveau de rats après injection par voie intraveineuse de nanoparticules d'oxyde de gadolinium

1,4 mL d'une solution injectable préparée selon l'exemple 5 ([Gd] = 5 mM) ont été injectés par voie intraveineuse dans une veine caudale d'un rat porteur d'une tumeur implantée selon l'exemple 6. Les images pondérées T₁ obtenues 20 minutes après injection intraveineuse des nanoparticules révèlent un faible rehaussement (blanchiment) dans la zone de la tumeur qui est cependant suffisamment important pour ne pas être ambigu (rehaussement du contraste : 15%). Le blanchiment résulte d'une concentration plus forte des nanoparticules dans la zone tumorale que dans le tissu sain. Une telle différence peut s'expliquer par une vascularisation de la tumeur plus importante que celle du tissu sain et/ou par une accumulation passive des particules dans la tumeur en raison d'une porosité accrue des vaisseaux sanguins irriguant les tumeurs.

La figure 5 montre les images pondérées T1 du cerveau d'un rat avant (figure 5a) et après (figure 5b) injection intraveineuse d'une solution injectable de nanoparticules d'oxyde de gadolinium selon cet exemple. Le blanchiment est repéré par une flèche.

### Exemple 10. Traitement des rats porteurs d'une tumeur par microfaisceaux X

Les rats porteurs d'une tumeur implantée selon l'exemple 6 ont été irradiés en mode MRT (microbeam radiation therapy) avec une dose à la peau de 625 Gy en tir unidirectionnel sur 51 microfaisceaux de 25 microns de largeur et espacés de 200 microns.

La figure 3 montre la courbe de survie de rats porteurs d'un gliosarcome (implanté selon l'exemple 6) ; la courbe I est obtenue pour les rats de l'exemple 6 (sans irradiation). La courbe II correspond aux rats traités selon le présent exemple (avec irradiation).

Les rats malades n'ayant pas été traités sont tous décédés 19 jours après l'implantation alors que les rats ayant été irradiés survivent plus longtemps.

Les rats ainsi traités survivent plus longtemps que les rats non irradiés comme le montre le déplacement de la courbe de survie de la figure 3 vers la droite. Cependant tous les rats décèdent moins de trente jours après l'implantation.

### Exemple 11. Traitement des rats porteurs d'une tumeur par microfaisceaux X après injection intratumorale d'une solution de chélates de gadolinium

Vingt minutes après l'injection intratumorale au centre de la tumeur (c'est-à-dire en réutilisant le site de trépanation utilisé au moment de l'injection des cellules) de 15 µL d'une solution commerciale de chélates de gadolinium ([Gd] = 500 mM), les rats porteurs d'une tumeur (implantée selon l'exemple 6) ont été irradiés en mode faisceaux croisés avec une dose à la peau de 460 Gy pour chaque faisceau des 51 microfaisceaux dont la largeur était de 25 µm et l'espacement de 200 µm. Le champ d'irradiation utilisé était de 13 x 10. Une nette amélioration de la survie est constatée puisque environ 33% des rats survivent 45 jours après l'implantation.

### Exemple 12 (comparatif). Traitement des rats porteurs d'une tumeur par microfaisceaux X après injection intratumorale d'une solution de nanoparticules d'or recouvertes de chélates de gadolinium (Au@DTDTPA-Gd)

Vingt minutes après l'injection intratumorale au centre de la tumeur (c'est-à-dire en réutilisant le site de trépanation utilisé au moment de l'injection des cellules) de 15 µL de solution injectable de nanoparticules d'or Au@DTDTPA-Gd préparée selon le protocole décrit par Debouttière et al. (Adv. Funct. Mater. 2006, 16, 2330-2339) ([Au] = 45mM et [Gd] = 5 mM), les rats porteurs d'une tumeur (implantée selon l'exemple 6) ont été irradiés en mode faisceaux croisés avec une dose à la peau de 460 Gy pour chaque faisceau des 51 microfaisceaux dont la largeur était de 25 µm et l'espacement de 200 µm. Le champ d'irradiation utilisé était de 13 x 10. Une amélioration de la survie est constatée puisque environ 25% des rats survivent 40 jours après l'implantation. Cependant tous les rats sont morts au-delà de 45 jours après l'implantation.

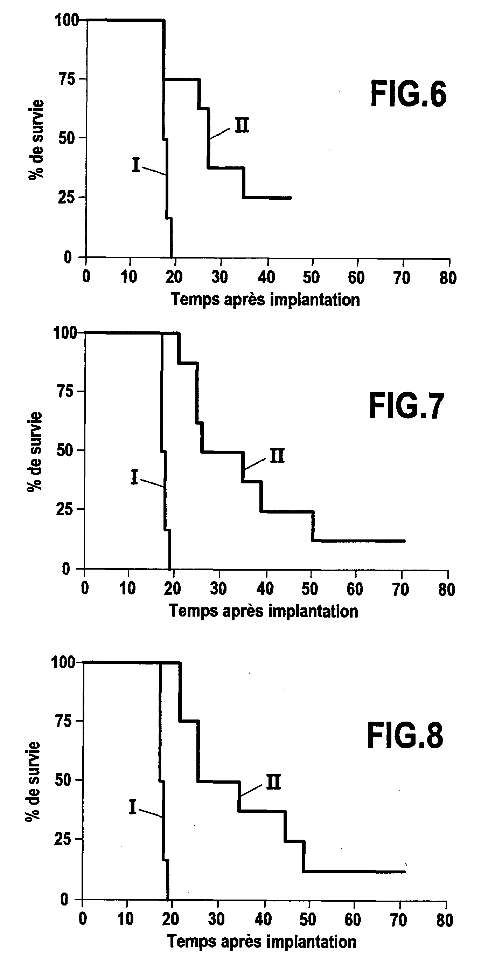

La Figure 6 montre la courbe de survie de rats porteurs d'un gliosarcome (implanté selon l'exemple 6) après injection intratumorale d'une solution de nanoparticules Au@DTDTPA-Gd (courbe II) et irradiation par microfaisceaux X (selon le présent exemple) par rapport aux témoins non traités (courbe I).

### Exemple 13 (comparatif). Traitement des rats porteurs d'une tumeur par microfaisceaux X après injection intraveineuse d'une solution de nanoparticules d'or recouvertes de chélates de gadolinium (Au@DTDTPA-Gd)

Vingt minutes après l'injection intraveineuse de 1,4 mL d'une solution injectable de nanoparticules d'or Au@DTDTPA-Gd préparée selon le protocole décrit par Debouttière et al. (Adv. Funct. Mater. 2006, 16, 2330-2339) ([Au] = 45mM et [Gd] = 5 mM) dans une de leurs veines caudales, les rats porteurs d'une tumeur (implantée selon l'exemple 6) ont été irradiés en mode faisceaux croisés avec une dose à la peau de 460 Gy pour chaque faisceau des 51 microfaisceaux dont la largeur était de 25 µm et l'espacement de 200 µm. Le champ d'irradiation utilisé était de 13 x 10. Une amélioration de la survie est constatée puisque environ 33% des rats survivent 45 jours après l'implantation. Cependant tous les rats sont morts au-delà de 50 jours après l'implantation.

### Exemple 14. Traitement des rats porteurs d'une tumeur par microfaisceaux X après injection intratumorale d'une solution de nanoparticules d'oxyde de gadolinium

Vingt minutes après l'injection intratumorale au centre de la tumeur (c'est-à-dire en réutilisant le site de trépanation utilisé au moment de l'injection des cellules) de 15 µL de solution injectable de nanoparticules d'oxyde de gadolinium préparée selon l'exemple 5 ([Gd] = 5 mM), les rats porteurs d'une tumeur (implantée selon l'exemple 6) ont été irradiés en mode faisceaux croisés avec une dose à la peau de 460 Gy pour chaque faisceau des 51 microfaisceaux dont la largeur était de 25 µm et l'espacement de 200 µm. Le champ d'irradiation utilisé était de 13 x 10. Une nette amélioration de la survie est constatée puisque environ 25% des rats survivent 45 jours après l'implantation (Figure 7).

### Exemple 15. Traitement des rats porteurs d'une tumeur par microfaisceaux X après injection intraveineuse d'une solution intraveineuse de nanoparticules d'oxyde de gadolinium

Vingt minutes après l'injection intraveineuse de 1,4 mL d'une solution injectable de nanoparticules d'oxyde de gadolinium préparée selon l'exemple 5 ([Gd] = 5 mM), les rats porteurs d'une tumeur (implantée selon l'exemple 6) ont été irradiés en mode faisceaux croisés avec une dose à la peau de 460 Gy pour chaque faisceau des 51 microfaisceaux dont la largeur était de 25 µm et l'espacement de 200 µm. Le champ d'irradiation utilisé était de 13 x 10. Une nette amélioration de la survie est constatée puisque environ 25% des rats survivent 45 jours après l'implantation.

La Figure 8 montre la courbe de survie de rats porteurs d'un gliosarcome (implanté selon l'exemple. 6) après injection intraveineuse d'une solution de nanoparticules d'oxyde de gadolinium et irradiation par microfaisceaux X, selon cet exemple (courbe II, à comparer avec la courbe I de survie sans traitement).

Remarque : Les exemples 10-15 montrent que le traitement avec les rayons X améliore la survie des animaux comparés aux animaux non traités (exemple 6). Cette amélioration est plus nette lorsque des agents radiosensibilisants sont administrés aux animaux malades. Il est important de constater que l'utilisation des nanoparticules d'oxyde de gadolinium enrobées par une couche de polysiloxane fonctionnalisée par du DTPA donne des résultats similaires à ceux des complexes de gadolinium dû vraisemblablement à une concentration locale des particules dans la zone à traiter plus importante alors même que les solutions de complexes sont 100 fois plus concentrées en gadolinium que les solutions de nanoparticules. Plus surprenant encore, l'effet des nanoparticules d'oxyde de gadolinium est supérieur à celui des particules d'or alors que l'élément or (neuf fois plus abondant dans les solutions injectées que le gadolinium) est caractérisé par un numéro atomique Z plus élevé. On notera que l'effet est même supérieur, compte tenu du fait que tous les rats qui ont été traités avec les nanoparticules d'or sont tous décédés après 45 à 50 jours alors qu'une partie des rats traités par les nanoparticules d'oxyde de gadolinium sont toujours vivants dans la même période.

## Revendications

1. Nanoparticules de dimensions comprises entre 1 et 50 nm, dont une partie au moins est constituée d'au moins un oxyde et/ou un oxohydroxyde d'au moins un lanthanide, lesdites nanoparticules étant :
- soit constituées exclusivement d'au moins un oxyde et/ou un oxohydroxyde d'au moins un lanthanide,
- soit sous forme de nanoparticules constituées exclusivement d'un coeur constitué exclusivement d'au moins un oxyde et/ou un oxohydroxyde d'au moins un lanthanide, et d'un enrobage constitué exclusivement d'un polysiloxane, avec éventuellement des molécules organiques greffées en surface ou comprises en son sein,
pour leur utilisation dans le traitement d'une tumeur, en tant qu'agent radio-sensibilisant dans une composition injectable utilisée pour améliorer l'efficacité du traitement d'une tumeur par irradiations X ou gamma.

2. Utilisation de nanoparticules de dimensions comprises entre 1 et 50 nm, dont une partie au moins est constituée d'au moins un oxyde et/ou un oxohydroxyde d'au moins un lanthanide, lesdites nanoparticules étant :
- soit constituées exclusivement d'au moins un oxyde et/ou un oxohydroxyde d'au moins un lanthanide,
- soit sous forme de nanoparticules constituées exclusivement d'un coeur constitué exclusivement d'au moins un oxyde et/ou un oxohydroxyde d'au moins un lanthanide, et d'un enrobage constitué exclusivement d'un polysiloxane, avec éventuellement des molécules organiques greffées en surface ou comprises en son sein,
en tant qu'agent radio-sensibilisant dans la fabrication d'une composition injectable destinée à améliorer l'efficacité du traitement d'une tumeur par irradiations X ou gamma.

3. Nanoparticules selon la revendication 1 ou utilisation selon la revendication 2, caractérisée(s) en ce que lesdites nanoparticules ont des dimensions inférieures à 5 nm, de préférence inférieures à 2 nm.

4. Nanoparticules selon la revendication 1 ou 3 ou utilisation selon la revendication 2 ou 3, caractérisée(s) en ce que lesdites nanoparticules contiennent des oxydes et/ou des oxohydroxydes d'au moins deux lanthanides différents, représentant chacun plus de 10 % en masse de la totalité des lanthanides et, de préférence, plus de 20 %.

5. Nanoparticules ou utilisation selon la revendication 4, caractérisée(s) en ce que les oxydes et/ou oxohydroxydes de lanthanides différents sont en couches successives.

6. Nanoparticules ou utilisation selon la revendication 4, caractérisée(s) en ce que les oxydes et/ou oxohydroxydes de lanthanides différents sont sous forme d'une solution solide.

7. Nanoparticules selon la revendication 1 ou l'une des revendications 3 à 6, ou utilisation selon l'une des revendications 2 à 6, caractérisée(s) en ce que lesdits lanthanides comprennent au moins 50 % en masse de lanthanides provoquant un signal en IRM, permettant une détection *in vivo* de la présence desdites particules et/ou un monitorage de la thérapie.

8. Nanoparticules selon la revendication 1 ou l'une des revendications 3 à 7, ou utilisation selon l'une des revendications 2 à 7, caractérisée(s) en ce que lesdits lanthanides contiennent au moins 50 % en masse de gadolinium (Gd), de dysprosium (Dy), d'holmium (Ho) ou de mélanges de ces lanthanides.

9. Nanoparticules selon la revendication 1 ou l'une des revendications 3 à 8, ou utilisation selon l'une des revendications 2 à 8, caractérisée(s) en ce qu'au moins une partie des lanthanides sont choisis pour avoir une section efficace de capture de neutrons suffisante pour permettre également un traitement par neutron-thérapie.

10. Nanoparticules selon la revendication 1 ou l'une des revendications 3 à 9, ou utilisation selon l'une des revendications 2 à 9, caractérisée(s) en ce que lesdits oxydes et/ou hydroxydes de lanthanides comprennent au moins 30 % en masse d'oxyde de luthétium, ou d'ytterbium ou d'un de leurs mélanges.

11. Nanoparticules selon la revendication 1 ou l'une des revendications 3 à 10, ou utilisation selon l'une des revendications 2 à 10, caractérisée(s) en ce que lesdits lanthanides comprennent au moins 50 % de gadolinium.

12. Nanoparticules selon la revendication 1 ou l'une des revendications 3 à 11, ou utilisation selon l'une des revendications 2 à 11, caractérisée(s) en ce que ladite partie contenant au moins un oxyde et/ou un oxohydroxyde d'au moins un lanthanide contient à sa périphérie des lanthanides provoquant un signal IRM, de préférence du gadolinium, et au moins un autre lanthanide dans sa partie centrale.

13. Nanoparticules selon la revendication 1 ou l'une des revendications 3 à 12, ou utilisation selon l'une des revendications 2 à 12, caractérisée(s) en ce que lesdites nanoparticules présentent un enrobage inorganique ou mixte inorganique/organique et en ce que lesdits oxydes et/ou oxohydroxydes de lanthanides représentent au moins 30 % en masse par rapport à l'ensemble des constituants inorganiques de ladite nanoparticule, les constituants organiques étant liés de façon covalente à la nanoparticule hybride et représentant moins de 30 % en masse de la particule finale.

14. Nanoparticules selon la revendication 1 ou l'une des revendications 3 à 13, ou utilisation selon l'une des revendications 2 à 13, caractérisée(s) en ce que lesdites nanoparticules comprennent un coeur sous forme d'au moins un oxyde et/ou un oxohydroxyde d'au moins un lanthanide et un enrobage en polysiloxane représentant 1 à 70 % en masse des constituants inorganiques de ladite nanoparticule.

15. Nanoparticules ou utilisation selon la revendication 14, caractérisée(s) en ce que ledit enrobage est sous forme d'un polysiloxane et en ce que le rapport nombre d'atomes de silicium/nombre d'atomes de lanthanides est compris entre 0,1 et 8.

16. Nanoparticules ou utilisation selon l'une des revendications 14 ou 15, caractérisée(s) en ce que ledit enrobage est un enrobage en polysiloxane et en ce que ladite nanoparticule comprend en outre des molécules organiques hydrophiles, de masses molaires inférieures à 5 000 g/mol et, de préférence inférieures à 450 g/mol, de préférence choisies parmi les molécules organiques comportant des fonctions alcools ou acides carboxyliques ou amines ou amides ou esters ou éther-oxydes ou sulfonates ou phosphonates ou phosphinates, liées de façon covalente à au moins 10 % des atomes de silicium dudit polysiloxane.

17. Nanoparticules ou utilisation selon la revendication 16, caractérisée(s) en ce que lesdites molécules organiques hydrophiles contiennent un motif polyéthylèneglycol, DTPA, DTDTPA (DTPA dithiolé) ou un acide succinique.

18. Nanoparticules ou utilisation selon la revendication 16 ou 17, caractérisée(s) en ce que lesdites molécules organiques hydrophiles sont des complexants des lanthanides dont la constante de complexation est supérieure à 10¹⁵, de préférence le DTPA ou le DOTA.

19. Nanoparticules selon la revendication 1 ou l'une des revendications 3 à 18, ou utilisation selon l'une des revendications 2 à 18, caractérisée(s) en ce que ladite composition contient entre 0,5 et 200 g/L d'oxyde(s) et/ou d'oxohydroxydes de lanthanides.

20. Nanoparticules selon la revendication 1 ou l'une des revendications 3 à 19, ou utilisation selon l'une des revendications 2 à 19, caractérisée(s) en ce que ladite composition est sous forme d'une dispersion colloïdale injectable.

## Patentansprüche

1. Nanopartikel mit Abmessungen zwischen 1 und 50 nm, von denen mindestens ein Teil von mindestens einem Oxid und/oder einem Oxohydroxid mindestens eines Lanthanids gebildet ist, wobei die Nanopartikel:
- entweder ausschließlich von mindestens einem Oxid und/oder einem Oxohydroxid mindestens eines Lanthanids gebildet sind,
- oder in Form von Nanopartikeln vorhanden sind, die ausschließlich von einem Kern, der ausschließlich von mindestens einem Oxid und/oder einem Oxohydroxid mindestens eines Lanthanids gebildet ist, und einer Ummantelung gebildet sind, die ausschließlich von einem Polysiloxan gebildet ist, eventuell mit organischen Molekülen, die an der Oberfläche aufgesetzt oder im Inneren enthalten sind,
für ihre Verwendung bei der Behandlung eines Tumors als radiosensibilisierender Wirkstoff in einer injizierbaren Zusammensetzung, die verwendet wird, um die Wirksamkeit der Behandlung eines Tumors durch Röntgen- oder Gammastrahlen zu verbessern.

2. Verwendung von Nanopartikeln mit Abmessungen zwischen 1 und 50 nm, von denen mindestens ein Teil von mindestens einem Oxid und/oder einem Oxohydroxid mindestens eines Lanthanids gebildet ist, wobei die Nanopartikel:
- entweder ausschließlich von mindestens einem Oxid und/oder einem Oxohydroxid mindestens eines Lanthanids gebildet sind,
- oder in Form von Nanopartikeln vorhanden sind, die ausschließlich von einem Kern, der ausschließlich von mindestens einem Oxid und/oder einem Oxohydroxid mindestens eines Lanthanids gebildet ist, und einer Ummantelung gebildet sind, die ausschließlich von einem Polysiloxan gebildet ist, eventuell mit organischen Molekülen, die an der Oberfläche aufgesetzt oder im Inneren enthalten sind,
als radiosensibilisierender Wirkstoff bei der Herstellung einer injizierbaren Zusammensetzung, die dazu bestimmt ist, die Wirksamkeit der Behandlung eines Tumors durch Röntgen- oder Gammastrahlen zu verbessern.

3. Nanopartikel nach Anspruch 1 oder Verwendung nach Anspruch 2, **dadurch gekennzeichnet, dass** die Nanopartikel Abmessungen unter 5 nm, vorzugsweise unter 2 nm, haben.

4. Nanopartikel nach Anspruch 1 oder 3 oder Verwendung nach Anspruch 2 oder 3, **dadurch gekennzeichnet, dass** die Nanopartikel Oxide und/oder Oxohydroxide mindestens zweier unterschiedlicher Lanthanide enthalten, die jeweils mehr als 10 Masse-% der Gesamtheit der Lanthanide aufweisen, und vorzugsweise mehr als 20 %.

5. Nanopartikel oder Verwendung nach Anspruch 4, **dadurch gekennzeichnet, dass** die Oxide und/oder Oxohydroxide von unterschiedlichen Lanthaniden in aufeinanderfolgenden Schichten vorhanden sind.

6. Nanopartikel oder Verwendung nach Anspruch 4, **dadurch gekennzeichnet, dass** die Oxide und/oder Oxohydroxide von unterschiedlichen Lanthaniden in Form einer festen Lösung vorhanden sind.

7. Nanopartikel nach Anspruch 1 oder einem der Ansprüche 3 bis 6, oder Verwendung nach einem der Ansprüche 2 bis 6, **dadurch gekennzeichnet, dass** die Lanthanide mindestens 50 Masse-% von Lanthaniden umfassen, die ein IRM-Signal hervorrufen und *in vivo* eine Erfassung des Vorhandenseins der Partikel und/oder ein Monitoring der Therapie ermöglichen.

8. Nanopartikel nach Anspruch 1 oder einem der Ansprüche 3 bis 7, oder Verwendung nach einem der Ansprüche 2 bis 7, **dadurch gekennzeichnet, dass** die Lanthanide mindestens 50 Masse-% Gadolinium (Gd), Dysprosium (Dy), Holmium (Ho) oder Gemische dieser Lanthanide enthalten.

9. Nanopartikel nach Anspruch 1 oder einem der Ansprüche 3 bis 8, oder Verwendung nach einem der Ansprüche 2 bis 8, **dadurch gekennzeichnet, dass** mindestens ein Teil der Lanthanide derart gewählt wird, dass er einen ausreichenden Einfangquerschnitt für Neutronen hat, um auch eine Behandlung durch Neutronen-Therapie zu ermöglichen.

10. Nanopartikel nach Anspruch 1 oder einem der Ansprüche 3 bis 9, oder Verwendung nach einem der Ansprüche 2 bis 9, **dadurch gekennzeichnet, dass** die Oxide und/oder Hydroxide von Lanthaniden mindestens 30 Masse-% von Lutheniumoxid oder Ytterbiumoxid oder eines ihrer Gemische umfassen.

11. Nanopartikel nach Anspruch 1 oder einem der Ansprüche 3 bis 10, oder Verwendung nach einem der Ansprüche 2 bis 10, **dadurch gekennzeichnet, dass** die Lanthanide mindestens 50 % Gadolinium umfassen.

12. Nanopartikel nach Anspruch 1 oder einem der Ansprüche 3 bis 11, oder Verwendung nach einem der Ansprüche 2 bis 11, **dadurch gekennzeichnet, dass** der Teil, der mindestens ein Oxid und/oder ein Oxohydroxid mindestens eines Lanthanids enthält, an seiner Peripherie Lanthanide enthält, die ein IRM-Signal hervorrufen, vorzugsweise Gadolinium, und mindestens ein weiteres Lanthanid in seinem zentralen Teil.

13. Nanopartikel nach Anspruch 1 oder einem der Ansprüche 3 bis 12, oder Verwendung nach einem der Ansprüche 2 bis 12, **dadurch gekennzeichnet, dass** die Nanopartikel eine anorganische oder gemischt anorganische/organische Ummantelung aufweisen, und dass die Oxide und/oder Oxohydroxide von Lanthaniden mindestens 30 Masse-% bezogen auf die Gesamtheit der anorganischen Bestandteile des Nanopartikels darstellen, wobei die organischen Bestandteile auf kovalente Weise mit dem Hybridnanopartikel verbunden sind und weniger als 30 Masse-% des endgültigen Partikels aufweisen.

14. Nanopartikel nach Anspruch 1 oder einem der Ansprüche 3 bis 13, oder Verwendung nach einem der Ansprüche 2 bis 13, **dadurch gekennzeichnet, dass** die Nanopartikel einen Kern in Form von mindestens einem Oxid und/oder einem Oxohydroxid mindestens eines Lanthanids und eine Ummantelung aus Polysiloxan umfassen, die 1 bis 70 Masse-% der anorganischen Bestandteile des Nanopartikels darstellt.

15. Nanopartikel oder Verwendung nach Anspruch 14, **dadurch gekennzeichnet, dass** die Ummantelung in Form eines Polysiloxans ist, und dass das Verhältnis Anzahl von Siliziumatomen/Anzahl von Lanthanidatomen zwischen 0,1 und 8 liegt.

16. Nanopartikel oder Verwendung nach einem der Ansprüche 14 oder 15, **dadurch gekennzeichnet, dass** die Ummantelung eine Ummantelung aus Polysiloxan ist, und dass der Nanopartikel ferner hydrophile organische Moleküle mit Molmassen unter 5000 g/mol und vorzugsweise unter 450 g/mol, umfasst, die vorzugsweise unter den organischen Molekülen, umfassend Alkoholfunktionen, oder Carboxylsäuren oder Aminen oder Amiden oder Estern oder Ätheroxiden oder Sulfonaten oder Phosphonaten oder Phosphinaten, die auf kovalente Weise mit mindestens 10 % der Siliziumatome des Polysiloxans verbunden sind, ausgewählt sind.

17. Nanopartikel oder Verwendung nach Anspruch 16, **dadurch gekennzeichnet, dass** die hydrophilen organischen Moleküle ein Polyethylenglykol-Motiv, DTPA, DTDTPA (Dithiol-DTPA) oder eine Bernsteinsäure enthalten.

18. Nanopartikel oder Verwendung nach Anspruch 16 oder 17, **dadurch gekennzeichnet, dass** die hydrophilen organischen Moleküle Komplexbildner der Lanthanide sind, deren Komplexbildungskonstante größer als 10¹⁵ ist, vorzugsweise DTPA oder DOTA.

19. Nanopartikel nach Anspruch 1 oder einem der Ansprüche 3 bis 18, oder Verwendung nach einem der Ansprüche 2 bis 18, **dadurch gekennzeichnet, dass** die Zusammensetzung zwischen 0,5 und 200 g/L Oxide und/oder Oxohydroxide von Lanthaniden enthält.

20. Nanopartikel nach Anspruch 1 oder einem der Ansprüche 3 bis 19, oder Verwendung nach einem der Ansprüche 2 bis 19, **dadurch gekennzeichnet, dass** die Zusammensetzung in Form einer injizierbaren kolloidalen Dispersion vorhanden ist.

## Claims

1. Nanoparticles with dimensions comprised between 1 and 50 nm, at least one portion of which consists of at least one oxide and/or one oxohydroxide of at least one lanthanide, said nanoparticles:
- either consisting exclusively of at least one oxide and/or one oxohydroxide of at least one lanthanide,
- or in the form of nanoparticles consisting exclusively of a core consisting exclusively of at least one oxide and/or one oxohydroxide of at least one lanthanide, and a coating consisting exclusively of a polysiloxane, with possibly organic molecules grafted at the surface or comprised inside it,
for their use in the treatment of a tumor as a radio-sensitizing agent in an injectable composition used for improving the efficiency of the treatment of a tumor by X or gamma irradiations.

2. The use of nanoparticles with dimensions comprised between 1 and 50 nm, at least one portion of which consists of at least one oxide and/or one oxohydroxide of at least one lanthanide, said nanoparticles:
- either consisting exclusively of at least one oxide and/or one oxohydroxide of at least one lanthanide,
- or in the form of nanoparticles consisting exclusively of a core consisting exclusively of at least one oxide and/or one oxohydroxide of at least one lanthanide, and a coating consisting exclusively of a polysiloxane, with possibly organic molecules grafted at the surface or comprised inside it,
as a radio-sensitizing agent in the making of an injectable composition intended to improve the efficiency of the treatment of a tumor by X or gamma irradiations.

3. The nanoparticles according to claim 1 or the use according to claim 2, **characterized in that** said nanoparticle has dimensions of less than 5 nm, preferably less than 2 nm.

4. The nanoparticles according to claim 1 or 3 or the use according to claim 2 or 3, **characterized in that** said nanoparticles contain oxides and/or oxohydroxides of at least two different lanthanides each accounting for more than 10% by mass of the totality of the lanthanides and preferably more than 20%.

5. The nanoparticles or the use according to claim 4, **characterized in that** the oxides and/or oxohydroxides of different lanthanides are in successive layers.

6. The nanoparticles or the use according to claim 4, **characterized in that** the oxides and/or oxohydroxides of different lanthanides are in the form of a solid solution.

7. The nanoparticles according to claim 1 or anyone of claims 3 to 6 or the use according to anyone of claims 2 to 6, **characterized in that** said lanthanides comprise at lest 50% by mass of lanthanides producing a signal in MRI, allowing *in vivo* detection of the presence of said particles and/or monitoring of the therapy.

8. The nanoparticles according to claim 1 or anyone of claims 3 to 7 or the use according to anyone of claims 2 to 7, **characterized in that** said lanthanides contain at least 50% by mass of gadolinium (Gd), of dysprosium (Dy), of holmium (Ho) or of mixtures of these lanthanides.

9. The nanoparticles according to claim 1 or anyone of claims 3 to 8 or the use according to anyone of claims 2 to 8, **characterized in that** at least one portion of the lanthanides is selected in order to have a sufficient cross-section for neutron capture so as to also allow treatment by neutron therapy.

10. The nanoparticles according to claim 1 or anyone of claims 3 to 9 or the use according to anyone of claims 2 to 9, **characterized in that** said oxides and/or hydroxides of lanthanides comprise at least 30% by mass of lutetium or ytterbium oxide, or of one of their mixtures.

11. The nanoparticles according to claim 1 or anyone of claims 3 to 10 or the use according to anyone of claims 2 to 10, **characterized in that** said lanthanides comprise at least 50% gadolinium.

12. The nanoparticles according to claim 1 or anyone of claims 3 to 11 or the use according to anyone of claims 2 to 11, **characterized in that** said portion containing at least one oxide and/or oxohydroxide of at least one lanthanide contains at its periphery lanthanides producing an MRI signal, preferably gadolinium and at least one other lanthanide in its central portion.

13. The nanoparticles according to claim 1 or anyone of claims 3 to 12 or the use according to anyone of claims 2 to 12, **characterized in that** said nanoparticles have an inorganic or mixed inorganic/organic coating and **in that** said oxides and/or oxohydroxides of lanthanides account for at least 30% by mass relatively to the whole of the inorganic constituents of said nanoparticle, the organic constituents being covalently bound to the hybrid nanoparticle and accounting for less than 30% by mass of the final particle.

14. The nanoparticles according to claim 1 or anyone of claims 3 to 13 or the use according to anyone of claims 2 to 13, **characterized in that** said nanoparticles comprise a core in the form of at least one oxide and/or oxohydroxide of at least one lanthanide and a polysiloxane coating accounting for 1 to 70% by mass of the inorganic constituents of said nanoparticle.

15. The nanoparticles or the use according to claim 14, **characterized in that** said coating is in the form of a polysiloxane and **in that** the silicon atom number/lanthanide atom number ratio is comprised between 0.1 and 8.

16. The nanoparticles or the use according to claim 14 or 15, **characterized in that** said coating is a polysiloxane coating and **in that** said nanoparticle further comprises hydrophilic organic molecules with molar masses of less than 5,000 g/mol and preferably less than 450 g/mol preferably selected from organic molecules including alcohol or carboxylic acid or amine or amide or ester or ether-oxide or sulfonate or phosphonate or phosphinate functions, covalently bound to at least 10% of the silicon atoms of said polysiloxane.

17. The nanoparticles or the use according to claim 16, **characterized in that** said hydrophilic organic molecules contain a polyethylene glycol, DTPA, DTDTPA (dithioly DTPA) unit or succinic acid.

18. The nanoparticles or the use according to claim 16 or 17, **characterized in that** said hydrophilic organic molecules are complexing agents of lanthanides, the complexation constant of which is greater then 10¹⁵, preferably DTPA or DOTA.

19. The nanoparticles according to claim 1 or anyone of claims 3 to 18 or the use according to anyone of claims 2 to 18, **characterized in that** said composition contains between 0.5 and 200 g/L of oxide(s) and/or oxohydroxides of lanthanides.

20. The nanoparticles according to claim 1 or anyone of claims 3 to 19 or the use according to anyone of claims 2 to 19, **characterized in that** said composition is in the form of an injectable colloidal dispersion.
